# EUROPEAN PATENT SPECIFICATION

(11) **EP 2 555 690 B1**
(45) Date of publication and mention of the grant of the patent: **02.07.2014**
(21) Application number: 11713641.6
(22) Date of filing: 01.04.2011
(51) Int. Cl.: A61B 17/12, A61B 17/00

(54) **VASO-OCCLUSIVE DEVICES**
VASO-OKKLUSIVE VORRICHTUNG
ELEMENT VASO-OCCLUSIF

(30) Priority: 05.04.2010 US 321052 P
(43) Date of publication of application: 13.02.2013
(73) Proprietor: Boston Scientific Scimed, Inc., Maple Grove, MN 55311-1566 (US)
(72) Inventor: CHEN, Hancun, San Ramon, California 94582 (US); MURPHY, Richard, Sunnyvale, California 94086 (US)
(74) Representative: Hauck Patent- und Rechtsanwälte
(86) International application number: PCT/US2011/030972
(87) International publication number: WO 2011/126946

(56) References cited:
- EP-A1- 0 824 011
- US-A- 5 690 667
- US-A1- 2009 053 281
- US-A1- 2009 270 908
- US-B1- 6 231 590

## Description

### FIELD

This application relates generally to devices for treating aneurysms, and more specifically, to vaso-occlusive devices.

### BACKGROUND

Vaso-occlusive devices are surgical implements or implants that are placed within the vasculature of the human body, typically via a catheter, either to block the flow of blood through a vessel making up that portion of the vasculature via the formation of an embolus or to form such an embolus within an aneurysm stemming from the vessel. One widely used vaso-occlusive device is provided in the form of a helical coil having "secondary shape" windings dimensioned to engage the walls of the vessels. Such vaso-occlusive coils are delivered out of a catheter to fill an aneurysm.

Applicants of the subject application determine that vaso-occlusive device formed using wire with larger outer diameter may be better for occupying the space of an aneurysm. However, using larger outer diameter wire to form the vaso-occlusive device may result in the vaso-occlusive device having too much stiffness, which may not be desirable for filling an aneurysm because the vaso-occlusive device may not be able to bend properly within the aneurysm.

From EP 0 824 011 A1 a flexible, vaso-occlusive device is known having an outside diameter less than about 0,254 mm (0.010 inches). The device shall be sufficiently flexible and small to be hydraulically delivered to a site within a vasculature of the human body.

From US 2009/270908 A1 an occluding device and a method of occluding fluid through a body vessel are known, wherein the occluding device comprises an inner coil and an outer coil disposed about a portion of the inner coil. The outer coil has a pre-curled tension to facilitate the outer coil to be curled within the lumen of the body vessel when deployed.

From US 6 231 590 B1 a medical device is known for forming an embolism within a vasculature of a patient. The vaso-occlusion device is at least partially coated with a bioactive agent, a collagenous material, or a collagenous coating optionally containing or coated with other bioactive agents.

From US 2009/053281 A1 (the preamble of claim 1 is based on this document) a vaso-occlusive device is known with a helically wounded elongated member having a core and an outer layer.

From US 5 690 667 A a vaso-occlusive helical metal coil is known having a thermoplastic polymer plug in at least one of its ends that has been melted so that it molds to the coil windings and insulates and blunts the end(s).

### SUMMARY

In accordance with some embodiments, a vaso-occlusive device includes a coil comprising a helically wounded elongated member having a core and an outer layer, wherein the outer layer is made from a metal or alloy having a stiffness that is different from a stiffness of the core.

Other and further embodiments and features will be evident from reading the following detailed description.

### BRIEF DESCRIPTION OF THE DRAWINGS

The drawings illustrate the design and utility of embodiments, in which similar elements are referred to by common reference numerals. These drawings are not necessarily drawn to scale. In order to better appreciate how the above-recited and other advantages and objects are obtained, a more particular description of the embodiments will be rendered, which are illustrated in the accompanying drawings.
**FIGS. 1-10** illustrate vaso-occlusive devices, some being constructed in accordance with various embodiments of the invention.
**FIG. 11** illustrates a vaso-occlusive device which may be constructed in accordance with still another embodiment of the invention, the vaso-occlusive device having a "C" shaped secondary configuration.
**FIG. 12** illustrates a vaso-occlusive device which may be constructed in accordance with yet another embodiment, the vaso-occlusive device having a clover-leaf secondary shape.
**FIG. 13** illustrates a vaso-occlusive device which may be constructed in accordance with still another embodiment, the vaso-occlusive device having a double-looped secondary shape.
**FIG. 14** illustrates an exemplary vaso-occlusive device which may be constructed according to one embodiment being delivered to an in-vivo site using a catheter.
**FIGS. 15A-15D** illustrate a procedure for introducing a vaso-occlusive device into an aneurysm.

### DESCRIPTION OF THE EMBODIMENTS

Various embodiments are described hereinafter with reference to the figures. It should be noted that the figures are not drawn to scale and that elements of similar structures or functions are represented by like reference numerals throughout the figures. It should also be noted that the figures are only intended to facilitate the description of the embodiments.

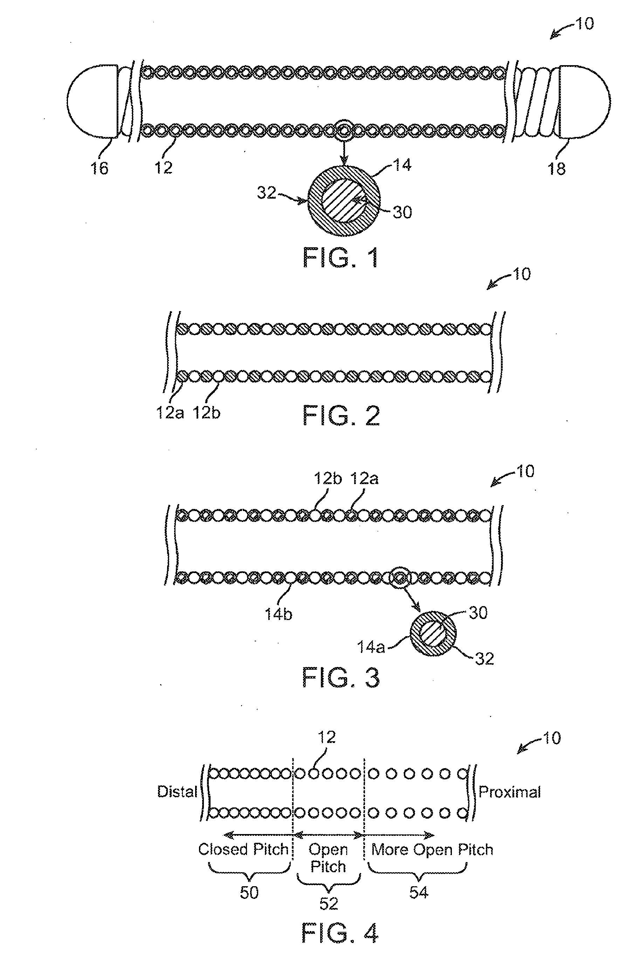

**FIG. 1** illustrates a vaso-occlusive device 10 in accordance with some embodiments. The vaso-occlusive device 10 includes a coil 12 formed by an elongated member 14, and has a distal end 16 and a proximal end 18. In the illustrated embodiments, the elongated member 14 making up the coil 12 is a wire, and has a core 30 and an outer layer 32. The core 30 may be made from a metal, such as pure platinum, and the outer layer 32 may be made from an alloy, such as platinum-tungsten alloy, e.g., 8% tungsten and the remainder platinum. The mechanical strength of pure platinum is about 1/3 of the platinum-tungsten alloy. Thus, the softer platinum core 30 may provide a softer coil 12 (e.g., for a given cross sectional dimension of the elongated member 14) and may provide memory property for shape retention.

It should be noted that the cross sectional dimension of the wire 14, and the sizes of the core 30 and outer layer 32 may be varied, depending on the requirements for a particular coil design. For example, if a stiffer coil 12 is desired, then the outer layer 32 may be made thicker while reducing the amount of material for the core 30 and keeping a constant cross sectional dimension for the wire 14. On the other hand, if a softer coil 12 is desired, then the core 30 may be made larger, while maintaining the same cross sectional dimension for the wire 14. In other embodiments, in addition to, or instead of, configuring the dimensions of the outer layer 32 and the core 30 to achieve a desirable stiffness for the coil 12, the materials for the core 30 and the outer layer 32 may be selected so that the desired stiffness for the coil 12 is achieved.

Also, it should be noted that any metallic materials may be used to form the core 30 and the outer layer 32 as long as they are biocompatible. In the above embodiments, the material for the core 30 is softer than the material for the outer layer 32. However, in other embodiments, the outer layer 32 may be made from a material that is softer (e.g., less stiff) than the material for the core 30. For example, in other embodiments, the outer layer 32 may be made from platinum, while the core 30 may be made from platinum-tungsten alloy, e.g., 8% tungsten and the remainder platinum.

**FIG. 2** illustrates another vaso-occlusive device 10. The vaso-occlusive device 10 includes a first coil 12a and a second coil 12b. The first and second coils 12a, 12b are axially aligned with each other, and are coupled to each other in a staggered configuration, wherein at least one loop from the first coil 12a is between two adjacent loops from the second coil 12b. In the illustrated device, the vaso-occlusive 10 may be formed by co-winding two elongated members (e.g., two wires).

In the illustrated device, the first coil 12a and the second coil 12b may be made from different materials. For example, in some devices, the first coil 12a may be made from a metal, such as pure platinum, and the second coil 12b may be made from an alloy, such as platinum-tungsten alloy, e.g., 8% tungsten and the remainder platinum. The mechanical strength of pure platinum is about 1/3 of the platinum-tungsten alloy. Thus, forming part of the device 10 using a softer wire may provide a softer vaso-occlusive device 10 (e.g., for a given cross sectional dimension of the elongated member forming the coil 12a/12b). It should be noted that the first coil 12a is not limited to being formed from a metal, and that in other embodiments, the first coil 12a may be made from an alloy or a non-metallic material. Also, in other devices, the second coil 12b may not be an alloy, and may be made from a metal or a non-metallic material.

As shown in FIG. 3, in other embodiments, the first coil 12a may be made from a wire having a core 30 and an outer layer 32, and the second coil 12b may be made from a metal (e.g., platinum), alloy (e.g., platinum-tungsten alloy), or a non-metallic material. In such embodiments, the core of the wire making up the first coil 12a may be made from metal, such as platinum, and the outer layer may be made from an alloy, such as platinum-tungsten alloy, e.g., 8% tungsten and the remainder platinum. The mechanical strength of pure platinum is about 1/3 of the platinum-tungsten alloy. Thus, the softer platinum core 30 may provide a softer coil 12a (e.g., for a given cross sectional dimension of the elongated member forming the coil 12a). This in turn provides a softer vaso-occlusive device 10.

In the embodiments, the core and the outer layer are made from different materials. For example, the materials for the core and the outer layer described previously may be switched. In the illustrated embodiments, the wires 14a, 14b forming the respective coils 12a, 12b have the same cross-sectional dimension. In other embodiments, the wires 14a, 14b forming the respective coils 12a, 12b may have different cross-sectional dimensions, which may be selected to achieve a desired stiffness for the vaso-occlusive device 10.

In any of the embodiments described herein, the vaso-occlusive device 10 may have coil with different degrees of pitch along the length of the coil 12. For example, as shown in **FIG. 4****,** the vaso-occlusive device 10 may have a closed pitch at a distal section 50, an open pitch at an intermediate section 52, and a more open pitch at a proximal section 54. Such configuration provides a variable stiffness along the length of the vaso-occlusive device 10, wherein the distal section 50 is stiffer than the proximal sections 52 and 54. In the configuration shown, the stiffer distal section 50 allows the vasoocclusive device to better anchor against the wall of an aneurysm. On the other hand, the softer proximal sections 52, 54 improve the coil softness, which may be better for packing inside the aneurysm because the proximal sections may be bent more easily.

In other embodiments, instead of having three sections with three different pitches, the vaso-occlusive device 10 may have more than three sections with more than three different pitches, or may have less than three sections with less than three different pitches. In further embodiments, the variability of the pitch along the length of the vaso-occlusive device 10 may be gradual.

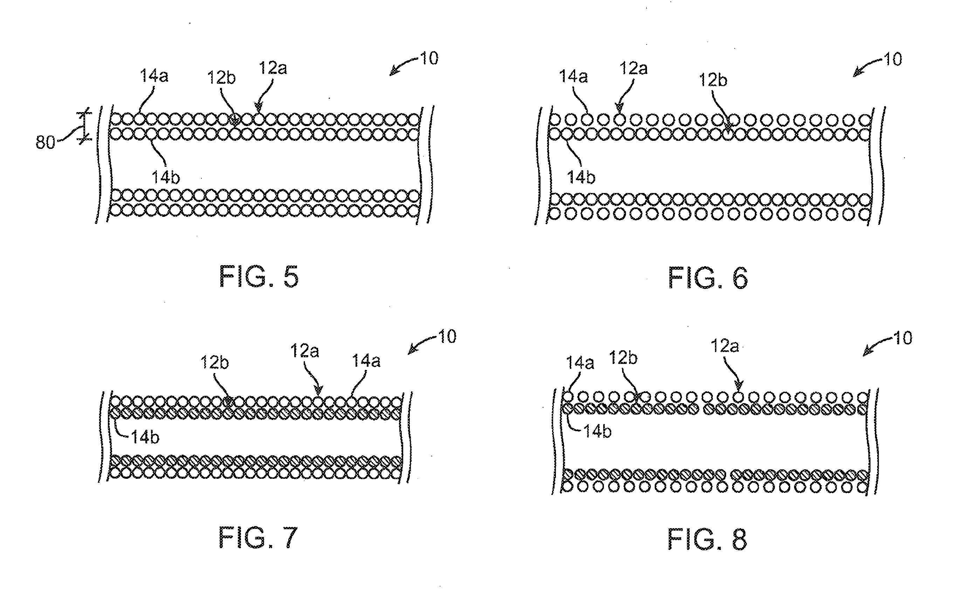

**FIG. 5** illustrates another vaso-occlusive device 10. The vaso-occlusive device 10 includes a first coil 12a and a second coil 12b. The first coil 12a has a plurality of loops that define a lumen therethrough, and the second coil 12b is located within the lumen of the first coil 12a. In the illustrated devices, the first coil 12a and the second coil 12b are made from the same material. For example, the first and second coils 12a, 12b may be made from a metal, such as pure platinum. In other devices, the coils 12a, 12b may be made from an alloy, such as platinum-tungsten alloy, e.g., 8% tungsten and the remainder platinum. In further devices, the coils 12a, 12b may be made from any material (e.g., metal, alloy, or a non-metallic material) as long as they are biocompatible. In the illustrated devices, the wires 14a, 14b that form the respective coils 12a, 12b have the same cross-sectional dimension. In other devices, the wires 14a, 14b that form the respective coils 12a, 12b may have different cross-sectional dimensions.

It should be noted that the vaso-occlusive device 10 of FIG. 5 is advantageous because forming the vaso-occlusive device 10 using two coils 12a, 12b may provide a softer vaso-occlusive device 10. In particular, the vaso-occlusive device 10 formed by the two coils 12a, 12b with a combined thickness 80 will be more flexible compared to a vaso-occlusive device formed from a single wire having a cross-sectional dimension 80, and with a same material as that of the coils 12a, 12b. In other words, for a given thickness 80 of a wall of the vaso-occlusive device 10, and a given material, formed the vaso-occlusive device 10 using two coils 12a, 12b will achieve a softer vaso-occlusive device that that formed using a single coil. This is because a single wire with a cross-sectional dimension 80 will provide a cross-sectional property (e.g., moment of inertia, or bending stiffness) that is higher than the cross-sectional property of the combined two wires 14a, 14b. Also, when the vaso-occlusive device 10 undergoes bending, part(s) of the coil 12b along the length of the coil 12b will be allowed to move (e.g., slide longitudinally) relative to the coil 12a, thereby allowing the vaso-occlusive device 10 to more easily bend.

**FIG. 6** illustrates a variation of the vaso-occlusive device 10 of **FIG. 5****.** In particular, the vaso-occlusive device is the same as that shown in **FIG. 5****,** except that the outer coil 12a has loops that are more spaced apart than that of the inner coil 12b. As shown in the illustrated devices, the inner coil 12b has a closed pitch, and the outer coil 12a has an open pitch. Such configuration may provide further softness for the vaso-occlusive device 10. In other devices, the inner coil 12b may also have an open pitch. In such devices, the outer coil 12a may have an open pitch that is more open that that of the inner coil 12b.

In further devices, the inner coil 12b may have a pitch that is more open that that of the outer coil 12a. For example, in other devices, the outer coil 12a may have a closed pitch, and the inner coil 12b may have an open pitch. Such configuration provides a vaso-occlusive device 10 that has a substantially continuous outer surface formed by the loops of the outer coil 12a.

In other devices, the first and second coils 12a, 12b in the devices 10 of FIGS. 5 and 6 may be made from different materials. For example, as shown in **FIG. 7****,** in other devices, the outer coil 12a may be made from a metal, such as pure platinum, and the inner coil 12b may be made from an alloy, such as platinum-tungsten alloy, e.g., 8% tungsten and the remainder platinum. In other devices, the outer coil 12a may be made from an alloy, or a non-metallic material. Also, in other devices, the inner coil 12b may be made from a metal or a non-metallic material. By selecting different materials for constructing the coils 12a, 12b, a desired stiffness may be accomplished for the vaso-occlusive device 10.

**FIG. 8** illustrates a variation of the vaso-occlusive device 10 of **FIG. 7****.** In particular, the vaso-occlusive device 10 is the same as that shown in **FIG.** 7, except that the outer coil 12a has loops that are more spaced apart than that of the inner coil 12b. As shown in the illustrated devices, the inner coil 12b has a closed pitch, and the outer coil 12a has an open pitch. Such configuration may provide further softness for the vaso-occlusive device 10. In other devices, the inner coil 12b may also have an open pitch. In such devices, the outer coil 12a may have an open pitch that is more open that that of the inner coil 12b.

In further devices, the inner coil 12b may have a pitch that is more open that that of the outer coil 12a. For example, in other devices, the outer coil 12a may have a closed pitch, and the inner coil 12b may have an open pitch. Such configuration provides a vaso-occlusive device 10 that has a substantially continuous outer surface formed by the loops of the outer coil 12a.

Regarding the above-described devices of **FIGS. 5-8**, in embodiments the wire used for the outer coil may alternatively be made from a pure platinum or platinum-tungsten alloy, and the wire for the inner coil may alternatively be made of a material consisting of a platinum core with an outer layer of platinum-tungsten alloy, or from a material consisting of a core of platinum-tungsten alloy and an outer layer of platinum. More alternatively, the wire for the outer coil can be made from a material consisting of a platinum core and an outer layer of platinum-tungsten alloy, or from a material consisting of a platinum-tungsten core and an outer layer of pure platinum, while the wire for inner coil can be made from pure platinum or platinum-tungsten alloy. Furthermore, the respective outer and inner coils of devices not being according to the invention can alternatively be made of a polymer, a ceramic, a bioactive material, or a combination of such materials. For example, a bioactive coating may be applied to one or both of the outer and inner metallic, polymeric and/or ceramic coils.

Furthermore, while the above-described embodiments of **FIGS. 5-8** are directed to double-coil embodiments, i.e., having an outer coil layer and an inner coil layer, one or more additional coil layers may be included in alternative embodiments for a total of three or more coil layers, in accordance with the inventive embodiments described herein. Such three-or-more coil layer embodiments would comprise an outer coil layer, and two or more inner coil layers.

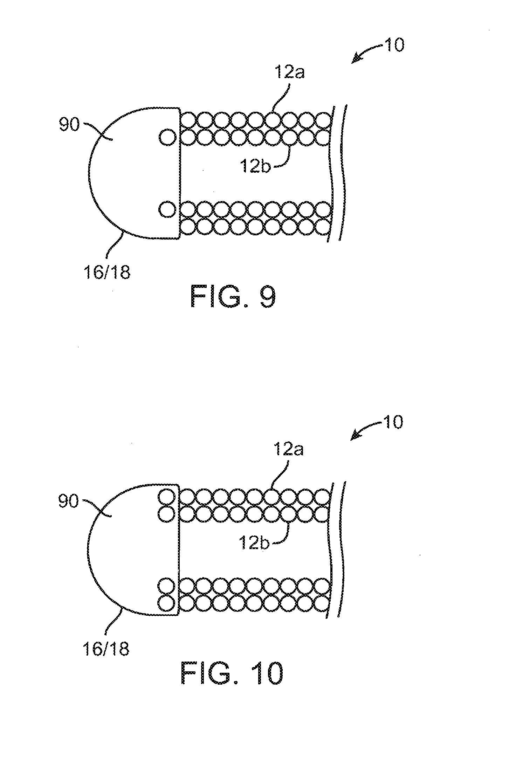

**FIG. 9** illustrates an end (e.g., a distal end 16 or a proximal end 18) of the vaso-occlusive device 10 in accordance with some embodiments. The vaso-occlusive device 10 may be any of the vaso-occlusive devices 10 described with reference to **FIGS. 5-8****.** As shown in the illustrated embodiments, the end of the inner coil 12b is fixedly attached to a blunt tip 90. In particular, the end of the inner coil 12b is embedded within the blunt tip 90. In other embodiments, the attachment of the coil 12b to the blunt tip 90 may be accomplished using a weld, glue, screw threads, or other suitable adhesive. In the illustrated embodiments, the outer coil 12a is coupled to the inner coil 12b by friction, and is not directly attached to the blunt tip 90. In other embodiments, the outer coil 12a may be directly secured to the inner coil 12b using a weld, glue, or a suitable adhesive. For example, the outer coil 12a may be welded to the inner coil 12b at one or both ends of the coil 12a. In further embodiments, in addition to, or instead of, securing to the inner coil 12b, the outer coil 12a may be directly attached to the blunt tip 90 **(****FIG. 10****).**

For example, the outer coil 12a may be embedded within the blunt tip 90 as that shown in the figure, or be directly attached to the blunt tip 90 using other mechanisms, such as a weld, glue, screw threads, or other suitable adhesive. In some embodiments, the inner coil 12b is secured to respective blunt tips at opposite ends of the inner coil 12b. The outer coil 12a may be secured to a blunt tip at one of the ends of the coil 12a, or to the respective blunt tips at both ends of the coil 12a. Securing the outer coil 12a to only one of the blunt tips (or to only one end of the inner coil 12b) has the advantage in that the outer coil 12a will be allowed to move more easily relative to the inner coil 12b, thereby providing a vaso-occlusive device 10 that is softer.

It should be noted that the materials for forming the coil(s) 12 of the vaso-occlusive device 10 should not be limited to the examples described previously. In any of the devices described herein, the material for the coil(s) 12 may be a radio-opaque material such as a metal or a polymer. The material for the coil(s) 12 may be rhodium, palladium, rhenium, as well as tungsten, gold, silver, tantalum, and alloys of these metals. These metals have significant radio-opacity and in their alloys may be tailored to accomplish an appropriate blend of flexibility and stiffness. They are also largely biologically inert. Also, any materials which maintain their shape despite being subjected to high stress may be used to construct the coil(s) 12.

For example, certain "super-elastic alloys" include various nickel/titanium alloys (48-58 atomic % nickel and optionally containing modest amounts of iron); copper/zinc alloys (38-42 weight % zinc); copper/zinc alloys containing 1-10 weight % of beryllium, silicon, tin, aluminum, or gallium; or nickel/aluminum alloys (36-38 atomic % aluminum), may be used. In further embodiments, titanium-nickel alloy known as "nitinol" may be used to form the coil(s) 12. These are very sturdy alloys which will tolerate significant flexing without deformation even when used as very small diameter wire.

In any of the embodiments described herein, the wire 14 used to form the coil may have a cross-sectional dimension that is in the range of 0,0005 mm and 0,15 mm (0.00002 and 0.006 inches). The coil 12 formed by the wire 14 may have a cross-sectional dimension between 0,076 mm and 0,64 mm (0.003 and 0.025 inches). For neurovascular applications, the coil 12 diameter may be anywhere from 0,2 mm to 0,46 mm (0.008 to 0.018 inches). In other embodiments, the wire 14 may have other cross-sectional dimensions, and the coil 12 may have other cross-sectional dimensions. In some embodiments, the wire 14 for forming the coil should have a sufficient diameter to provide a hoop strength to the resulting device 10 sufficient to hold the device 10 in place within the chosen body site, lumen or cavity, without substantially distending the wall of the site and without moving from the site as a result of the repetitive fluid pulsing found in the vascular system.

In any of the embodiments described herein, the axial length of the coil 12 may be in the range of 0.5 to 100 cm, and more preferably, in the range of 2.0 to 40 cm. Depending upon use, the coil 12 may have 10-75 turns per centimeter, or more preferably 10-40 turns per centimeter. In other embodiments, the coil 12 may have other lengths and/or other number of turns per centimeter.

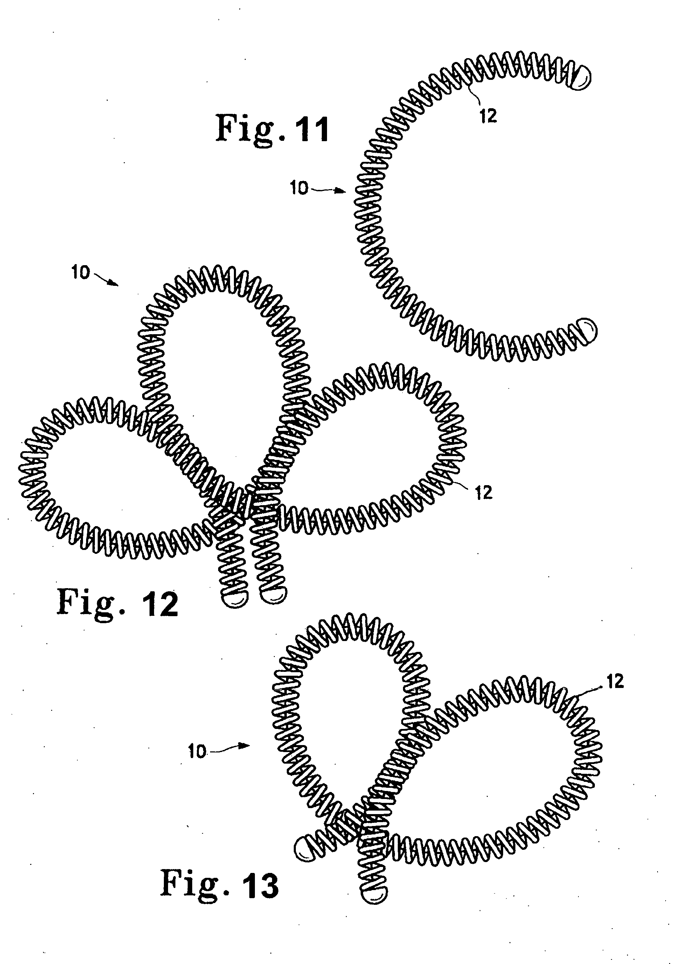

In some embodiments, the vaso-occlusive devices 10 described herein may have the simple linear shape shown previously, or may have shapes which are more complex. **FIGS. 11, 12,** and **13** show what are termed "secondary" shapes in that they are formed from the primary coil by winding the primary coil on a form of a desired shape and then heat treating the soformed shape. **FIG. 11** shows a vaso-occlusive device 10 having a "C" shape. **FIG. 12** shows a vaso-occlusive device 10 having a clover-leaf shape. **FIG. 13** shows a vaso-occlusive device 10 having a double-loop configuration. These are examples of the various secondary shapes that may be implemented in embodiments of the vaso-occlusive device 10 described herein.

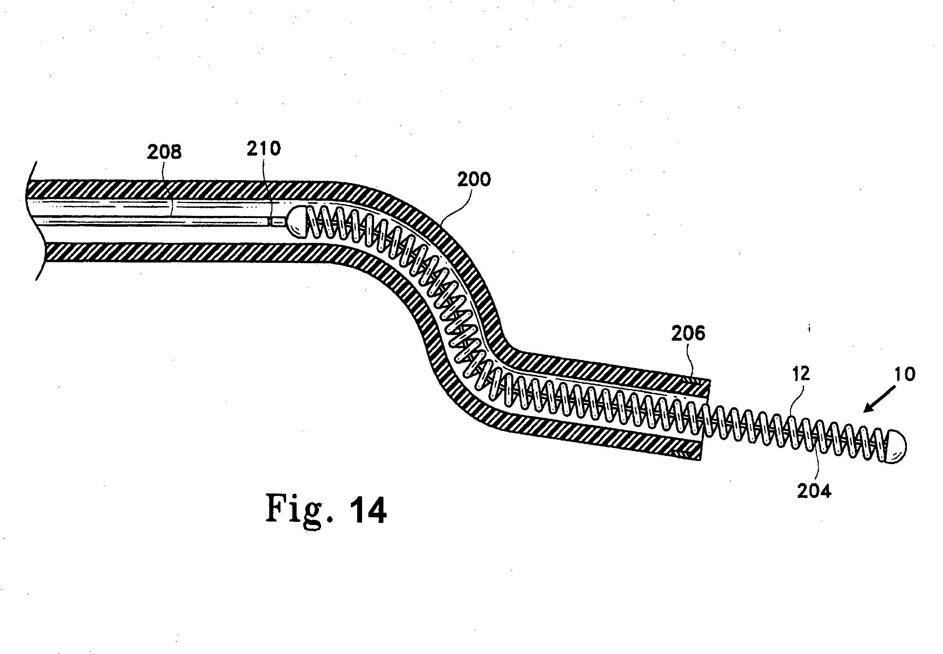

In any of the embodiments described herein, the vaso-occlusive device 10 may be delivered using a catheter. **FIG. 14** shows a catheter 200 containing the vaso-occlusive device 10 as described herein. The catheter 200 may have a distal radio-opaque marker 206 if so desired, so that a user may determine the positioning of the distal end of the catheter 200 within a body during use. Proximally of the vaso-occlusive device 10 is a connective wire 208 which is insulated at all points proximal of an electrolytic joint 210. The wire 208 may insulated with a combination of polytetrafluoroethylene and PARYLENE (polyparaxyxylene), except for the small sacrificial joint 210 which is intended to be the site of the electrolysis as the joint 210 is eroded or severed and the vaso-occlusive device 10 deployed into the body site.

In some embodiments, the distal portion of the catheter 200 may be made from any material, such as polyurethane, polyvinylchloride, silicones, or other polymers. The connective wire 208 used therein should be very flexible so that it does not interfere with the movement of the catheter 200. It is conductive and insulated proximally of the electrolytic joint 210. Introduction of an electric current into the connective wire 208 will cause the electrolytic joint 210 to erode and the vaso-occlusive device 10 to become detached. In the illustrated embodiments, the vaso-occlusive device 10 further includes a stretch-resisting member 204 for preventing over-stretching of the vaso-occlusive device 10. In other embodiments, the stretch-resisting member 204 is not needed.

It should be noted that the manner of releasing the vaso-occlusive device 10 from a delivery catheter is not limited to the example described, and that other ways of discharging vaso-occlusive devices into the human vasculature are possible. For example, in other embodiments, mechanically detachable devices may be used to detachably couple the vaso-occlusive device 10 to the pusher.

In some embodiments, the vaso-occlusive device 10 may be unscrewed from a pusher having interlocking surfaces. In other embodiments, interlocking clasps mounted both on the pusher and on the vaso-occlusive device 10 may be used to detachably couple the vaso-occlusive device 10 to the pusher. In other embodiments, an interlocking ball and keyway-type coupling may be used to detachably couple the vaso-occlusive device 10 from the pusher. In further embodiments, a pusher-vaso-occlusive coil assembly may have a proximally extending wire carrying a ball on its proximal end and a pusher having a similar end. The two ends are interlocked and disengage when expelled from the distal tip of the catheter.

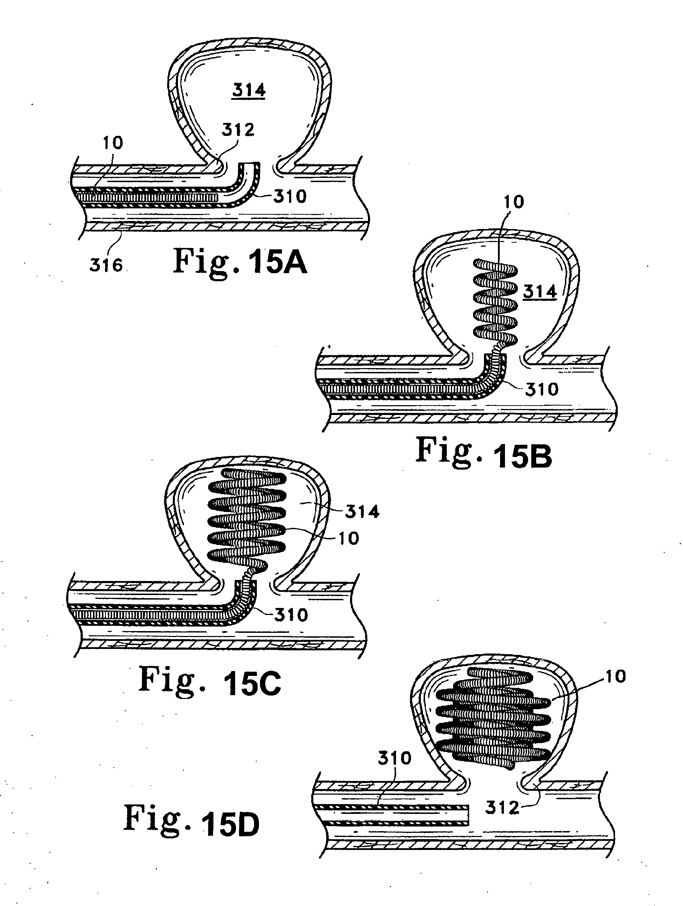

**FIGS. 15A-15D** depict a deployment method for introduction of any of the embodiments of the vaso-occlusive device 10 described here. Specifically, **FIG. 15A** shows the distal tip of a delivery catheter 310, which is within the opening 312 of an aneurysm 314 found in an artery 316. The distal or end section of the vaso-occlusive device 10 is shown within the catheter 310. The vaso-occlusive device 10 may be any of the vaso-occlusive devices described herein. The advancement of the vaso-occlusive device 10 relative to the catheter 310 may be achieved using a plunger/pusher that pushes the vaso-occlusive device 10 distally relative to the catheter 310.

In some embodiments, the vaso-occlusive device 10 may have a secondary shape. In such embodiments, while the vaso-occlusive device 10 is housed within the catheter 310, the vaso-occlusive device 10 is stretched into a low profile. When the vaso-occlusive device 10 is deployed out of the lumen of the catheter 310, the vaso-occlusive device 10 then assumes its relaxed configuration to have the secondary shape.

In **FIG. 15B****,** the distal end portion of the vaso-occlusive device 10 has exited the distal end of the catheter 310 and has wound into a secondary shape within the aneurysm 314. **FIG. 15C** shows the completion of the formation of the secondary shape within the aneurysm 314.

In some embodiments, the pusher merely abuts against the vaso-occlusive device 10. In such embodiments, as soon as the vaso-occlusive device 10 is deployed out of the lumen of the catheter 310, the vaso-occlusive device 10 is then separated from the pusher. In other embodiments, the vaso-occlusive device 10 may be mechanically coupled to the pusher. In such embodiments, the user may operate on the coupling mechanism to detach the vaso-occlusive device 10 from the pusher. For example, if the coupling mechanism includes an electrolytic joint, the user may apply energy to sever the joint, thereby releasing the vaso-occlusive device 10 from the pusher.

**FIG. 15D** shows the separation of the vaso-occlusive device 10 from the pusher, placement within the aneurysm 314, and the withdrawal of the catheter from the mouth of the aneurysm.

In any of the embodiments described herein, the vaso-occlusive device may further include fibrous elements coupled to the coil. For example, the fibrous elements may extend in a sinusoidal fashion down the length of the coil, and may be coupled to loops of the coil. The fibrous elements may enhance the ability of the coil to fill space within the vasculature and to facilitate formation of embolus and subsequent allied tissue. The fibrous materials may be made from biocompatible materials, such as Dacron (polyester), polyglycolic acid, polylactic acid, fluoropolymers (polytetrafluoroethylene), Nylon (polyamide), or silk. The strands forming the braid should be reasonably heavy, e.g., having tensile strength of greater than about 0,67 N (0.15 pounds). The materials mentioned, to the extent that they are thermoplastics, may be melted or fused to the coils. Alternatively, they may be glued or otherwise fastened to the coils. Preferred materials include Dacron.

Also, in any of the embodiments described herein, the vaso-occlusive device 10 may further include a stretch-resisting member coupled (e.g., soldered, brazed, glued, or otherwise fixedly attached) to both ends of the vaso-occlusive device 10 and extending through a central lumen of the vaso-occlusive device 10. The stretch-resisting member enhances the tensile strength of the vaso-occlusive device 10, and may prevent the vaso-occlusive device 10 from being over-stretched during use. In other embodiments, the stretch-resisting member may be coupled to the vaso-occlusive device 10 at one or more locations intermediate to the ends of the vaso-occlusive device 10. The stretch-resisting member may be thermoplastic or thermosetting and comprise a bundle of threads or a single filament melted onto, glued, or otherwise fixedly attached to the vaso-occlusive device 10. In some instances, it may also be desirable to include one or more metallic strands in the stretch-resisting member to provide stiffness or electrical conductance for specific applications.

## Claims

1. A vaso-occlusive device (10), comprising:
a coil (12) having a proximal end (18) and a distal end (16), the coil (12) comprising a helically wound elongated member (14) having a core (30) and an outer layer (32),
**characterized in that** the outer layer (32) being made from a metal or alloy, and has a stiffness that is different from a stiffness of the core (30).

2. The vaso-occlusive device (10) of claim 1, wherein the core (30) is made from a first material, and the outer layer (32) is made from a second material stiffer than the first material.

3. The vaso-occlusive device (10) of claim 1, wherein the core (30) is made from a first material, and the outer layer (32) is made from a second material softer than the first material.

4. The vaso-occlusive device (10) of claim 1, wherein the core (30) comprises platinum, and wherein the outer layer (32) comprises a platinum-tungsten alloy.

5. The vaso-occlusive device (10) of any of claims 1-4, wherein the coil (12) has a distal portion (50) and a proximal portion (54), and a pitch of the coil (12) at the distal portion (50) is less open than a pitch of the coil (12) at the proximal portion (54).

## Patentansprüche

1. Vaso-okklusive Vorrichtung (10), umfassend:
eine Wicklung (12), die ein proximales Ende (18) und ein distales Ende (16) aufweist, wobei die Wicklung (12) ein schraubenförmig gewickeltes längliches Element (14) umfasst, das einen Kern (30) und eine Außenschicht (32) aufweist,
**dadurch gekennzeichnet, dass** die Außenschicht (32) aus einem Metall oder einer Legierung hergestellt ist und eine Steifigkeit aufweist, die sich von einer Steifigkeit des Kerns (30) unterscheidet.

2. Vaso-okklusive Vorrichtung (10) nach Anspruch 1, wobei der Kern (30) aus einem ersten Werkstoff hergestellt ist und die Außenschicht (32) aus einem zweiten Werkstoff hergestellt ist, der steifer als der erste Werkstoff ist.

3. Vaso-okklusive Vorrichtung (10) nach Anspruch 1, wobei der Kern (30) aus einem ersten Werkstoff hergestellt ist und die Außenschicht (32) aus einem zweiten Werkstoff hergestellt ist, der weicher als der erste Werkstoff ist.

4. Vaso-okklusive Vorrichtung (10) nach Anspruch 1, wobei der Kern (30) Platin umfasst und wobei die Außenschicht (32) eine Platin-Wolfram-Legierung umfasst.

5. Vaso-okklusive Vorrichtung (10) nach einem der Ansprüche 1 - 4, wobei die Wicklung (12) einen distalen Abschnitt (50) und einen proximalen Abschnitt (54) aufweist, und ein Schritt der Wicklung (12) am distalen Abschnitt (50) weniger offen ist als ein Schritt der Wicklung (12) am proximalen Abschnitt.

## Revendications

1. Élément vaso-occlusif (10) comprenant :
une bobine (12) présentant une extrémité proximale (18) et une extrémité distale (16), la bobine (12) comprenant un élément allongé enroulé hélicoïdalement (14) présentant un noyau (30) et une couche extérieure (32),
**caractérisé en ce que** la couche extérieure (32) est constituée d'un métal ou d'un alliage et présente une rigidité différente de la rigidité du noyau (30).

2. Élément vaso-occlusif (10) selon la revendication 1, dans lequel le noyau (30) est constitué d'un premier matériau et la couche extérieure (32) est constituée d'un deuxième matériau plus rigide que le premier matériau.

3. Élément vaso-occlusif (10) selon la revendication 1, dans lequel le noyau (30) est constitué d'un premier matériau et la couche extérieure (32) est constituée d'un deuxième matériau plus souple que le premier matériau.

4. Élément vaso-occlusif (10) selon la revendication 1, dans lequel le noyau (30) comprend du platine et dans lequel la couche extérieure (32) comprend un alliage de platine et de tungstène.

5. Élément vaso-occlusif (10) selon l'une quelconque des revendications 1-4, dans lequel la bobine (12) présente une partie distale (50) et une partie proximale (54), et un pas de la bobine (12) dans la partie distale (50) est moins ouvert qu'un pas de la bobine (12) dans la partie proximale (54).
